(19) Europäisches Patentamt
European Patent Office
Office européen des brevets

(11) **EP 2 749 267 A1**

(12) **EUROPEAN PATENT APPLICATION**

(43) Date of publication:
02.07.2014 Bulletin 2014/27

(51) Int Cl.:
*A61K 9/00* (2006.01)          *A61K 31/357* (2006.01)
*A61K 47/44* (2006.01)          *A61P 33/06* (2006.01)

(21) Application number: 14161340.6

(22) Date of filing: 27.10.2008

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: 25.10.2007 GB 0720967
10.04.2008 GB 0806510

(62) Document number(s) of the earlier application(s) in accordance with Art. 76 EPC:
**08806806.9 / 2 209 464**

(71) Applicant: **Protopharma Limited**
**Norfolk NR4 7GJ (GB)**

(72) Inventors:
• **Booles, Clive**
**Norwich, Norfolk NR4 7GJ (GB)**
• **Ross, Calvin John**
**deceased (GB)**
• **Sams, Martin**
**Norwich, NR4 7GJ (GB)**

(74) Representative: **Harris, Oliver John Richard**
**Novagraaf UK**
**12 Meridian Way**
**Meridian Business Park**
**Norwich NR7 0TA (GB)**

Remarks:
This application was filed on 24-03-2014 as a divisional application to the application mentioned under INID code 62.

(54) **Anti-malarial pharmaceutical composition**

(57)     The invention provides pharmaceutical compositions for the treatment and prophylaxis of malaria, comprising artemether and a medium chain triglyceride formulated for transmucosal sublingual, buccal or nasal delivery, especially by a spray. Also provided are delivery devices containing the compositions.

Figure 1

Artemether Mean Concentration (ng/mL) vs Time (hour)

**Description**

Field of the Invention

The invention relates to pharmaceutical compositions, delivery methods, delivery devices and methods for the treatment of uncomplicated and complicated malaria.

Background and Prior Art Known to the Applicant

Malaria is an infectious disease widespread in many tropical and subtropical regions, caused by the infectious parasite *Plasmodium* transmitted primarily by the female mosquito of the *Anopheles* genus. Malaria is the cause of between one and three million deaths annually, mostly in sub-Saharan Africa. Of these, some 75% are of children under five.

Many pharmaceuticals have been developed or trialled for the treatment or prevention of malaria in both children and adults. Although useful pharmaceutical agents exist, and the life cycle of the malaria-carrying mosquito is well understood, practical intervention strategies have so far failed to bring this disease under control. As with most infectious diseases, issues of drug resistance are ever-present. However, for malaria, other confounding factors include the difficulty of administration of drugs to those in need, especially to children. In the most severely affected regions, children are often undernourished and suffer from other ailments. Apart from the symptoms caused by the malaria infection itself, episodes of diarrhoea and vomiting are not uncommon in such children. As a result, children are unable to swallow medicines in tablet form and it is extremely difficult to find appropriate veins in children for administration by the intravenous route. Even if this were possible, in many cases there are no trained medical personnel on hand to administer drugs intravenously, especially where a course of medication is required over a period of days or weeks.

Amongst the active pharmaceuticals of use in the treatment of malaria are a number of compounds derived from artemesenin, a sesquiterpene lactone endoperoxide originally isolated from *Artemesia annua* (Woodrow et al. Postgrad. Med.J. 2005; 81:71-78). These compounds include the semi-synthetic derivatives artenimol, artesunate, artemether and arteether (artemotil). The International Pharmacopoeia (*Ph. Int.,* World Health Organisation) lists a number of these for the treatment of malaria, viz: Artemether in the form of capsules, tablets or an injectable formulation; Artemesenin in the form of capsules or tablets; arteether in an injectable formulation; and both artenimol and artesunate in the form of tablets.

US Patent 6306896 describes pharmaceutically active compositions containing artemisinine and/or derivatives of artemisinine. The active ingredients are formulated for rectal administration, in the form of suppositories. Rectal administration of antimalarial therapeutics is particularly problematic, for a number of reasons: Firstly, many people suffering with malaria experience diarrhoea, making administration difficult. Secondly, for effective absorption through the rectal mucosa, patients need to have good nutritional status and a good diet, containing a high fat content; this is rarely the case in sub-Saharan Africa. Thirdly, in many communities affected by malaria, there are strong cultural barriers to the use and administration of suppositories.

It can be seen that all of these formulations face the difficulties of administration described above. It is therefore amongst the objects of the present invention to address these and other issues.

Summary of the Invention

Accordingly, the invention provides, in a first aspect, a pharmaceutical composition comprising: artemether or arteether; and a pharmaceutically-acceptable excipient selected the group consisting of: medium chain length triglycerides; short chain triglycerides; omega-3-marine triglycerides and fish oil, rich in omega-3-acids, said composition formulated for transmucosal sublingual, buccal or nasal dosage.

The inventors have found that the transmucosal sub-lingual, transmucosal buccal and transmucosal nasal routes for administration of artemether or arteether are effective for delivery of the pharmaceutical into the systemic circulation e.g. for the treatment of malaria. Furthermore, for the first time, it provides an administration route that is acceptable to children requiring treatment, and that may be administered by non-medically qualified personnel. It has particular advantage, therefore, in more remote village settings, where e.g. village elders can be trained in the diagnosis of malaria, and subsequent administration of the drug. The composition can be delivered e.g. sublingually as a liquid bolus, or, more preferably, as a spray.

Medium chain length triglycerides are defined in the European Pharmacopoeia Monograph 0868, as:

A mixture of triglycerides of saturated fatty acids, mainly of caprylic acid (octanoic acid, $C_8H_{16}O_2$) and of capric acid (decanoic acid, $C_{10}H_{20}O_2$). Medium-chain triglycerides are obtained from the oil extracted from the hard, dried fraction of the endosperm of *Cocos nucifera* L. or from the dried endosperm of *Elaeis guineensis* Jacq. When Medium-chain Triglycerides are prepared from the endosperm of *Cocos nucifera* L., the title Fractionated Coconut Oil may be used. Medium chain length triglycerides have a minimum 95.0 per cent of saturated fatty acids with 8 and 10 carbon atoms. Further chemical and physical properties are described in the European Pharmacopoeia Monograph 0868, and equivalent documents.

Short chain triglycerides are triglycerides having chain lengths of less than 6 carbon atoms.

Omega-3-marine triglycerides are defined in the European Pharmacopoeia Monograph 0868 as mixture of mono-, di- and triesters of omega-3 acids with glycerol containing mainly triesters and obtained either by esterification of concentrated and purified omega-3 acids with glycerol or by transesterification of the omega-3 acid ethyl esters with glycerol. The origin of the omega-3 acids is the body oil from fatty fish species coming from families like *Engraulidae, Carangidae, Clupeidae, Osmeridae, Salmonidae* and *Scombridae.* The omega-3 acids are identified as the following acids: alpha-linolenic acid (C18:3 n-3), moroctic acid (C18:4 n-3), eicosatetraenoic acid (C20:4 n-3), timnodonic (eicosapentaenoic) acid (C20:5 n-3; EPA), heneicosapentaenoic acid (C21:5 n-3), clupanodonic acid (C22:5 n-3) and cervonic (docosahexaenoic) acid (C22:6 n-3; DHA). The sum of the contents of the omega-3 acids EPA and DHA, expressed as triglycerides is a minimum of 45.0 per cent, and the total omega-3 acids, expressed as triglycerides is a minimum of 60.0 per cent. Tocopherol may be added as an antioxidant.

Fish oil, rich in omega-3-acids is also defined in the European Pharmacopeia as purified, winterised and deodorised fatty oil obtained from fish of the families *Engraulidae, Carangidae, Clupeidae, Osmeridae, Scombridae* and *Ammodytidae.* The omega-3 acids are defined as the following acids: alpha-linolenic acid (C18:3 n-3), moroctic acid (C18:4 n-3), eicosatetraenoic acid (C20:4 n-3), timnodonic (eicosapentaenoic) acid (C20:5 n-3; EPA), heneicosapentaenoic acid (C21:5 n-3), clupanodonic acid (C22:5 n-3) and cervonic (docosahexaenoic) acid (C22:6 n-3; DHA).

The content of the Fish oil, rich in omega-3-acids is as follows:

EPA, expressed as triglycerides: minimum 13.0 per cent,
DHA, expressed as triglycerides: minimum 9.0 per cent,
Total omega-3-acids, expressed as triglycerides: minimum 28.0 per cent.

Authorized antioxidants in concentrations not exceeding the levels specified by the competent authorities may be added.

Whilst these definitions serve to define particularly preferred compositions of the recited excipients, the skilled addressee will appreciate that the composition of appropriate alternative excipients may also deviate from these exact compositional limits. Excipients of choice should exhibit analogous chemical properties such as the ability to solubilise artemether or arteether at the required concentration, not to degrade the pharmaceutically active ingredients, and to be non-toxic. The excipients should also have analogous physical properties such as at least being liquid at body temperature, and preferably having a suitable viscosity to allow the excipient to be used in preferred spray formulations described below. The viscosity for these applications should be low enough to be capable of atomizing, as described below, when used in a pump spray.

As an example, compositions might consist essentially of artemether or arteether and a pharmaceutically acceptable excipient consisting essentially of a triglyceride, liquid at 37°C, and medium chain triglycerides (as defined herein).

Particularly preferred compositions of the invention consist essentially of: artemether or arteether; and one or more pharmaceutically-acceptable excipients selected the group consisting of: medium chain length triglycerides; short chain triglycerides; and omega-3-marine triglycerides, said composition formulated for transmucosal sublingual, buccal or nasal dosage. The exclusion of significant amounts of other materials (e.g. higher molecular weight lipids) renders a composition that is ideally suited to transmucosal nasal, buccal, and especially sublingual delivery.

More preferred compositions comprise: artemether and a pharmaceutically-acceptable excipient selected the group consisting of: medium chain length triglycerides; short chain triglycerides; and omega-3-marine triglycerides, said composition formulated for transmucosal sublingual, buccal or nasal dosage, and especially a composition consisting essentially of: artemether and a pharmaceutically-acceptable excipient selected the group consisting of: medium chain length triglycerides; short chain triglycerides; and omega-3-marine triglycerides, said composition formulated for transmucosal sublingual, buccal or nasal dosage.

In any of these compositions, it is especially preferred that the composition is substantially free of water, as the inventors have found, contrary to accepted belief, that water can significantly reduce the shelf-life of the compositions, especially when stored at ambient temperatures. Preferred compositions would have less than 1%(w/w) water, and more preferably less than 0.5%(w/w) water, and most preferably less than 0.1%(w/w) water.

Also in any of these compositions, it is especially preferred that the composition is substantially free of ethanol. Again, the inventors have found that ethanol leads to degradation of the pharmaceutically active components. Preferred compositions in particular have less than 1%(w/w) ethanol, and more preferably less than 0.5%(w/w) ethanol and most preferably less than 0.1%(w/w) ethanol.

Also in any of these compositions, it is preferred that artemether or arteether is present at a concentration of between 2 and 250 milligrams per gram of excipient. This concentration provides an appropriate level for the expected volumes used for the described transmucosal delivery. More preferably, the composition comprises: artemether or arteether, dissolved in the excipient at a concentration of between 2 and 200 milligrams per gram of excipient. Other preferred concentrations are between 2 and 100 milligrams per gram; between 2 and 50 milligrams per gram. The lower concentrations provide compositions particularly suitable for paediatric use, and are also more likely to ensure that the pharmaceutically active components remain in solution over a wide temperature range, rather than having some portion as e.g. a suspension. This is particularly important to ensure that delivery of the drug is by the recited transmucosal route. If significant amounts of the active components are not in solution, then there is an increased likelihood that some will be swallowed, thereby reducing the beneficial effects of such transmucosal delivery described below.

In especially preferred compositions, the said excipient comprises a medium chain triglyceride, said triglyceride comprising a minimum of 95 per cent of saturated fatty acids with between 6 and 12 carbon atoms. More preferably, said excipient comprises a medium chain triglyceride, said triglyceride comprising a minimum of 95 per cent of saturated fatty acids with between 8 and 10 carbon atoms.

Also in any such composition, it is also particularly preferred that the composition further comprises an essential oil such as menthol, vanillin or orange oil, lemon oil, clove oil, peppermint oil, spearmint oil. Particular technical advantages of such an essential oil, especially menthol, which acts as a solubilising agent, are described further below.

In a second aspect, the invention provides a medicament delivery device containing a composition described herein, said device adapted to deliver individual or successive doses of said composition, each individual or successive dose having a volume of less than 1000 microlitres. The use of small dose volumes reduces the likelihood that the composition will be swallowed, or spat out, by the patient. The likelihood is reduced further by use of smaller volumes (especially in the paediatric context or for nasal delivery) and so in further preferred embodiments, each successive dose has a volume of less than 600 microlitres; less than 400 microlitres; less than 200 microlitres; or even less than 100 microlitres. Smaller volumes are especially preferred for paediatric use, or nasal delivery.

In a third aspect, the invention provides a medicament delivery device containing a composition described herein, said device and composition adapted to deliver individual or successive doses of said composition, each individual or successive dose containing no more than 80mg of artemether or arteether. Such devices are preferably adapted to assist sublingual delivery, especially by non-medically trained personnel. Limiting the amount of active pharmaceutical delivered with each dose is especially important in the context of malaria treatment by less skilled personnel to ensure that over-dosing is avoided. Preferably, said device and composition adapted to deliver individual or successive doses of said composition, each individual or successive dose containing no more than 10mg of artemether or arteether. This provides an appropriate device for paediatric use.

Preferably, the delivery devices according to these aspects comprise a spray, and especially a pump spray. The use of a pump spray increases the area of mucosa to which the composition is applied, thereby increasing absorption and minimising the likelihood that the medicament is swallowed. More preferably, said device is adapted to produce a spray of composition having a mean droplet diameter greater than 20 microns, or even greater than 50 microns, or preferably greater than 75 microns. In this way, inadvertent delivery of the medicament to the lungs is avoided, or reduced.

In a fourth aspect, the invention also provides a device for providing pharmaceutical doses comprising a container containing a pharmaceutical composition described herein, and valve means arranged to transfer doses of said pharmaceutical composition to the exterior of the container. Such a device may be attached to e.g. a separate transmucosal buccal, nasal or sublingual delivery device, such as a spray.

In a fifth aspect, the invention provides a kit for the treatment or prophylaxis of malaria comprising a composition described herein and instructions to administer said composition to a patient in need thereof by the transmucosal sublingual, buccal or nasal route. Preferably, said kit has instructions to administer said composition to a patient in need thereof by the sublingual route.

In a sixth aspect, the invention provides a method of treating a disease responsive to artemether or arteether (and preferably artemether) comprising the administration to a patient in need thereof of a therapeutically effective amount of artemether or arteether by the transmucosal sublingual, buccal or nasal route. More preferably, said administration is by the sublingual route, and most preferably said disease is malaria.

The invention also provides a kit for the treatment of malaria comprising a composition described herein and instructions to administer said composition to a patient in need thereof by the transmucosal buccal or nasal, or more preferably sublingual route.

Also included in the scope of the invention is the use of artemether in the preparation of a pharmaceutical composition according to any of the aspects, or preferred aspects, described above for the treatment or prophylaxis of malaria.

Also included within the scope of the invention is a method of treating malaria comprising the administration to a patient in need thereof of a therapeutically effective amount of artemether in the form of a composition as described herein by the transmucosal sublingual, buccal or nasal route, preferably in the form of a spray.

Preferably, any of the pharmaceutical compositions or devices provided by the present invention are for the prophylaxis, or especially the treatment of malaria.

Also included within the scope of the invention are pharmaceutical compositions, medicament delivery devices, kits and methods substantially as described herein, with reference to, and as illustrated by any appropriate combination of the accompanying drawings.

Description and Preferred Embodiments of the Invention

One of the most important aspects of providing a clinically useful treatment for malaria is to provide a formulation and an administration route for any active ingredient that can withstand the challenges of those communities where malaria is an especially acute problem, such as in sub-Saharan Africa. For example, any formulation needs to be stable for long periods of time, and at the relatively high temperatures encountered there. The medicament will often need to be administered (without delay) to children who are weak, malnourished, and likely to be suffering from vomiting and diarrhoea. In many cases, the medicament may also need to be administered by non-medically-trained personnel. It is also important for any active ingredient to have good (and consistent) bioavailability, to ensure that the drug reaches the site of action without adverse side effects.

In order to address these problems, the inventors have found that the transmucosal sublingual, buccal or nasal route of administration of artemether provides a greater likelihood of higher and more reproducible levels of bioavailability than that demonstrated by the oral (i.e. swallowed) or intramuscular route. Navaratnam et al (Clin Pharmacokinet, 2000, Oct; 39(4): 255-270) report the bioavailability of artemether in animals by oral administration to be as low as 19-35%, and only 54% when administered by intramuscular injection. In humans, the bioavailability of artemether was low in both the intramuscular (25%) and intrarectal (35%) route, with considerable variability in absorption. The authors report that *"Preliminary studies in children with cerebral malaria indicated that the bioavailability of intramuscular artemether is highly variable and could potentially affect treatment outcome in the most severely ill patients ".*

The use of the transmucosal sublingual, buccal or nasal route of administration avoids the first-pass effect that occurs with oral and rectal administration. Whilst adults might be able to tolerate the large oral doses of artemether required to overcome the low bioavailability of the drug, this is not the case in children, and so the compositions disclosed herein are particularly suitable for the treatment of malarial in children, i.e. for paediatric formulations.

Preliminary results of initial, confidential, dose ranging studies are presented below, indicating surprisingly increased bioavailability of the drug when administered by sublingual spray in comparison to oral administration by tablet.

The inventors have also found that, contrary to accepted belief, artemether is not stable when in contact with water, ethanol, or propellants that might be used for aerosol formulations.

Tables 1 and 2 show impurities present in Artemether API, and artemether in three solvent systems: 20% ethanol + 80% propellant; 50% ethanol + 50% propellant; 100% ethanol; and a medium chain triglyceride, in this case, the triglyceride sold under the registered trade mark Miglyol® 810. Miglyol® is a medium chain triglyceride containing saturated C8 and C10 fatty acids, typically between 65-80% of caprylic acid (C8:0) and 20-35% of capric acid (C10:0).

The propellant used in these test was 1,1,1,2 tetrafluoroethane, sold under the registered trade mark Zephex® 134a. Similar results were obtained for the propellants butane, Zephex® 227 (1,1,1,2,3,3,3 heptafluoropropane) and for a mixture of butane and propane.

Table 1 shows the impurities (as a percentage of the peak area of an HPLC chromatogram of artemether) after storage of the compositions at 30°C for eight weeks. Table 2 shows the corresponding impurities after storage for eight weeks at 40°C.

*Table 1 - Storage at 30°C*

| Relative Retention Time: | 0.35 | 0.68 | 0.73 | 0.87 | 0.91 | 1.17 |
|---|---|---|---|---|---|---|
| | % of artemether | | | | | |
| Artemether API | 0.4 | | 0.1 | 0.2 | | |
| 20% EtOH 80% propellant | 1.6 | 0.3 | 0.7 | 0.2 | 1.3 | 0.2 |
| 50% EtOH 50% propellant | 1.0 | 0.2 | 0.5 | 0.2 | 1.5 | 0.2 |
| 100% EtOH | 0.3 | 0.2 | 0.5 | 0.2 | | |
| Miglyol 810® | 0.4 | | 0.1 | 0.2 | | |

*Table 2 - Storage at 40°C*

| Relative Retention Time: | 0.35 | 0.68 | 0.73 | 0.87 | 0.91 | 1.17 |
|---|---|---|---|---|---|---|
| | % of artemether | | | | | |
| Artemether API | 0.4 | | 0.1 | 0.2 | | |
| 20% EtOH 80% propellant | 4.9 | 1.9 | 2.9 | 0.2 | 5.3 | 1.4 |
| 50% EtOH 50% propellant | 2.2 | 1.4 | 2.5 | 0.2 | 4.8 | 1.0 |
| 100% EtOH | 2.2 | 0.7 | 1.6 | 0.2 | 1.0 | 0.7 |
| Miglyol 810® | 0.6 | | 0.1 | 0.2 | | |

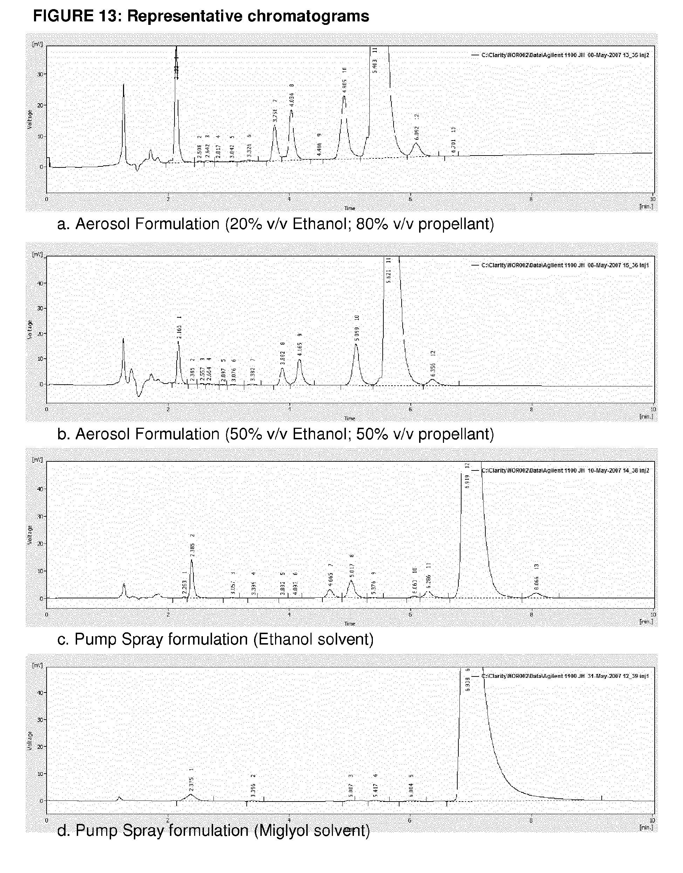

Representative chromatograms are shown in Figure 13. It can be seen that the levels of impurities in the Miglyol® 810 formulation are not significantly higher than those observed in the initial Artemether API. In all other cases, the impurities are at levels that exceed those permitted under the ICH Harmonised Tripartite Guidelines for Impurities in New Drug Products without specific identification or further toxicological examination.

A solution in a medium chain triglyceride, especially a saturated triglyceride such as Miglyol® 810 therefore constitutes a stable formulation for the active ingredient. Being a saturated triglyceride, it is believed that this confers stability to the artemether. Given its chemical structure, it is likely that the main route of degradation of artemether is via reduction mechanisms, which might explain the protection afforded by such saturated fatty acid-containing triglycerides.

When used in a spray delivery system, e.g. in a manually-actuated pump spray, the triglyceride also acts as a pump and valve lubricant, thereby removing the need to add additional lubricants to the formulation. The use of such medium chain triglycerides also produces a formulation of appropriate viscosity and surface tension for use in a pump spray delivery system.

Further advantages also flow from the use of medium chain triglyceride: being hydrophobic, the triglyceride adheres to the mucosa of the mouth, and so allows time for the artemether to be absorbed transmucosally. The hydrophobic nature of the composition resists being washed out of the mouth by the action of saliva, which would otherwise cause the active ingredient to be swallowed.

In especially preferred embodiments of the invention, the artemether-triglyceride solution is supplemented with menthol, or alternatively with orange oil or vanilla. The inventors have found that this has a number of benefits:

(1) Its function as a taste-masking agent is particularly important in the context of administration of anti-malarial drugs to children; the drug is often administered over a number of days, such as a three to five-day regime, and any bad taste of the drug experienced by the child on the first dose, makes it difficult to administer subsequent doses.

(2) The essential oil also acts as a penetration enhancer to improve the uptake of the pharmaceutical ingredient through the mucosa of the mouth.

(3) The addition of a flavour also allows the person administering the drug to check firstly that the drug has been dispensed (the patient can taste or smell it) and secondly that it has been dispensed into the right place - if the drug were e.g. accidentally dispensed directly into the throat, there would be no taste sensation.

(4) A surprising feature is that the essential oil (especially levomenthol) also assists with the solubilisation of the artemether. In a solubility trial, dissolution of artemether in miglyol occurred after 4 minutes 30 seconds when menthol added before artemether compared to 5 minutes 55 seconds when artemether added before menthol.

Preferred formulations (for sublingual or buccal paediatric use) are given in Tables 3 and 4. Two different dose concentrations are given suitable for use in a spray delivery system. A number of sprays (i.e. individual spray actuations of 100microlitres) may be given, dependent on the weight of the child to be treated:

*Table* 3: *3mg Artemether per actuation*

| Raw Material Item | Weight (g) | % w/w |
|---|---|---|
| Artemether IP | 0.090 | 3.2 |
| Levomenthol *Ph. Eur.* | 0.020 | 0.7 |
| Miglyol® 810 | 2.690 | 96.1 |

*Table 4: 6mg Artemether per actuation*

| Raw Material Item | Weight (g) | % w/w |
|---|---|---|
| Artemether IP | 0.180 | 6.4 |
| Levomenthol *Ph. Eur.* | 0.020 | 0.7 |
| Miglyol® 810 | 2.600 | 92.9 |

Table 5 outlines an example of a preferred dosage regime for paediatric use. Alternative regimes are envisaged, e.g. dosing at 3mg/kg body weight.

*Table 5: Paediatric Dosage Regime*

| Weight of child (kg) | Number of doses at 3mg Dose per spray actuation | Total delivered dose mg/kg | Number of doses at 6mg Dose per spray actuation | Total delivered dose mg/kg |
|---|---|---|---|---|
| 3 | 1 | 1.00 | | |
| 4 | 1 | 0.75 | | |
| 5 | 2 | 1.20 | | |
| 6 | 2 | 1.00 | | |
| 7 | 2 | 0.86 | | |
| 8 | 3 | 1.13 | | |
| 9 | 3 | 1.00 | | |
| 10 | 3 | 0.90 | | |
| 11 | 4 | 1.09 | | |
| 12 | 4 | 1.00 | 2 | 1.00 |
| 13 | 4 | 0.92 | 2 | 0.92 |
| 14 | 5 | 1.07 | 2 | 0.86 |
| 15 | 5 | 1.00 | 3 | 1.20 |
| 16 | 5 | 0.94 | 3 | 1.13 |
| 17 | | | 3 | 1.06 |
| 18 | | | 3 | 1.00 |
| 19 | | | 3 | 0.95 |
| 20 | | | 3 | 0.90 |
| 21 | | | 3 | 0.86 |
| 22 | | | 4 | 1.09 |
| 23 | | | 4 | 1.04 |
| 24 | | | 4 | 1.00 |
| 25 | | | 4 | 0.96 |
| 26 | | | 4 | 0.92 |
| 27 | | | 4 | 0.89 |
| 28 | | | 5 | 1.07 |
| 29 | | | 5 | 1.03 |
| 30 | | | 5 | 1.00 |

Formulations for adult use may be prepared at higher concentrations of artemether, such as 150-200 mg/ml. For adult use, individual spray volumes may be larger than the 100microlitre example described here for paediatric use.

Bioavailability of Artemether

The applicant has carried out confidential trials to asses the uptake of the artemether-containing compositions of the present invention when delivered by the sublingual route, by comparison to oral administration by tablet.
Trials were carried out on healthy male adult human volunteers (16 subjects per cohort), and subject to normal ethical approval. Three single-dose regimes according to the present invention were studied, and compared to a regime using oral-dosed tablets, as follows:

Sub-Lingual Spray Regimes

Spray formulations of artemether were prepared as detailed above, and administered, on a single occasion, to a group of volunteers by the sublingual route. A number of successive actuations of the spray were administered, as shown in Table 6, below.

*Table 6 - Dosage Regime for Single Dose Study Sublingual Spray Formulation*

| Test | Formulation | Dose per Actuation (mg) | Number of Actuations | Total Doge (mg) |
|------|-------------|-------------------------|----------------------|-----------------|
| T1 | As Table 3 | 3 | 5 | 15 |
| T2 | As Table 3 | 3 | 10 | 30 |
| T3 | As Table 4 | 6 | 5 | 30 |

Reference Oral Dose

As a reference, a fourth group of volunteers were administered tablets containing artemether, on a single occasion, as shown in Table 7, below.

*Table 7 - Dosage Regime for Single Dose Study Oral Tablet Formulation*

| Test | Formulation | Dose per Tablet (mg) | Number of Tablets | Total Doge (mg) |
|------|-------------|----------------------|-------------------|-----------------|
| T4 | Tablet | 10 | 3 | 30 |

Following administration of each dosage regime, blood samples were taken from the subjects, and plasma concentrations of artemether and its immediate metabolite dihydroartemesinin were determined, in order to compare bioavailability by the two routes.

Figures 1-6 show mean plasma concentration of artemether following two comparison dose regimes. Figures 7-12 show the corresponding mean plasma concentration of dihydroartemesinin.

Figures 1 and 7 compare regimes T1 (open squares) and T4 (closed circles): 15mg artemether via 5 sublingual spray doses vs. 30mg artemether via tablet.

Figures 2 and 8 compare regimes T2 (open squares) and T4 (closed circles): 30mg artemether via 10 sublingual spray doses vs. 30mg artemether via tablet.

Figures 3 and 9 compare regimes T3 (open squares) and T4 (closed circles): 30mg artemether via 5 sublingual spray doses vs. 30mg artemether via tablet.

Figures 4 and 10 compare regimes T1 (open squares) and T2 (closed circles): 15mg artemether via 5 sublingual spray doses vs. 30mg artemether via 10 sublingual spray doses.

Figures 5 and 11 compare regimes T2 (open squares) and T3 (closed circles): 30mg artemether via 10 sublingual spray doses vs. 30mg artemether via 5 sublingual spray doses.

Figures 6 and 12 compare regimes T1 (open squares) and T3 (closed circles): 15mg artemether via 5 sublingual spray doses vs. 30mg artemether via 5 sublingual spray doses).

Pharmacokinetic data for each of the four dosage regimes are given in Tables 8-11, below:

Table 8: Test Group T1

| Single sublingual administration of 15mg Artemether sublingual spray: 3mg per actuation | | |
|---|---|---|
| | Plasma Artemether | Plasma Dihydroartemesinin |
| Pharmacokinetic Parameters* | (n=16) (mean $\pm$SD) | (n=16) (mean $\pm$SD) |
| $AUC_{0-12}$ (ng.h/mL) | 25.85 $\pm$ 13.88 | 29.63 $\pm$ 11.58 |
| $C_{max}$ (ng/mL) | 16.11 $\pm$ 8.69 | 18.29 $\pm$ 7.52 |
| $T_{max}$ (h) | 1.70 $\pm$ 0.68 | 1.83 $\pm$ 0.68 |
| $t_{1/2}$ (h) | 0.72 $\pm$ 0.30 | |
| $\lambda_z$ (h$^{-1}$) | 1.11 $\pm$0.40 | |
| CL/F (ng/h) | 0.74 $\pm$ 0.46 | 0.54 $\pm$ 0.15 |
| V/F (L) | 0.68 $\pm$ 0.33 | 0.51 $\pm$ 0.16 |
| *Key: <br> $AUC_{0-12}$ (ng.h/mL) Area under the concentration curve between 0-12 h. <br> $C_{max}$ (ng/mL) Maximum observed plasma concentration <br> $T_{max}$ (h) Time of observed maximum plasma concentration <br> $t_{1/2}$ (h) Elimination half-life <br> $\lambda_z$ (h$^{-1}$) Elimination rate constant <br> CL/F (ng/h) Apparent clearance rate <br> V/F (L) Apparent volume of distribution | | |

Table 9: Test Group T2

| Single sublingual administration of 30mg Artemether sublingual spray: 3mg per actuation | | |
|---|---|---|
| | Plasma Artemether | Plasma Dihydroartemesinin |
| Pharmacokinetic Parameters | (n=16) (mean $\pm$SD) | (n=16) (mean $\pm$SD) |
| $AUC_{0-12}$ (ng.h/mL) | 76.60 $\pm$ 43.12 | 99.51 $\pm$ 50.33 |
| $C_{max}$ (ng/mL) | 32.12 $\pm$ 16.39 | 44.11 $\pm$ 28.48 |
| $T_{max}$ (h) | 1.73 $\pm$ 0.82 | 2.10 $\pm$ 1.17 |
| $t_{1/2}$ (h) | 1.39 $\pm$ 0.49 | |
| $\lambda_z$ (h$^{-1}$) | 0.56 $\pm$ 0.20 | |
| CL/F (ng/h) | 0.56 $\pm$ 0.37 | 0.36 $\pm$ 0.13 |
| V/F (L) | 1.00 $\pm$ 0.55 | 0.72 $\pm$ 0.36 |
| Key as Table 8 | | |

Table 10: Test Group T3

| Single sublingual administration of 30mg Artemether sublingual spray: 6mg per actuation | | |
|---|---|---|
| | Plasma Artemether | Plasma Dihydroartemesinin |
| Pharmacokinetic Parameters | (n=16) (mean $\pm$SD) | (n=16) (mean $\pm$SD) |
| $AUC_{0-12}$ (ng.h/mL) | 71.11 $\pm$ 41.08 | 86.19 $\pm$ 27.68 |
| $C_{max}$ (ng/mL) | 35.24 $\pm$ 23.91 | 41.14 $\pm$ 16.45 |

(continued)

| Single sublingual administration of 30mg Artemether sublingual spray: 6mg per actuation | | |
|---|---|---|
| | Plasma Artemether | Plasma Dihydroartemesinin |
| Pharmacokinetic Parameters | (n=16) (mean $\pm$ SD) | (n=16) (mean $\pm$ SD) |
| $T_{max}$ (h) | 1.67 $\pm$ 0.77 | 1.88 $\pm$ 0.74 |
| $t_{1/2}$ (h) | 1.40 $\pm$ 0.59 | |
| $\lambda_z$ (h$^{-1}$) | 0.59 $\pm$ 0.25 | |
| CL/F (ng/h) | 0.63 $\pm$ 0.49 | 0.39 $\pm$ 0.15 |
| V/F (L) | 1.01 $\pm$ 0.49 | 0.91 $\pm$ 0.67 |
| Key as Table 8 | | |

Table 11: Test Group T4

| Single oral administration of 30mg Artemether Tablets 10mg per Tablet | | |
|---|---|---|
| | Plasma Artemether | Plasma Dihydroartemesinin |
| Pharmacokinetic Parameters | (n=16) (mean $\pm$ SD) | (n=16) (mean $\pm$ SD) |
| $AUC_{0-12}$ (ng.h/mL) | 34.59 $\pm$ 21.01 | 38.49 $\pm$ 12.38 |
| $C_{max}$ (ng/mL) | 10.12 $\pm$ 7.19 | 10.99 $\pm$ 4.39 |
| $T_{max}$ (h) | 1.02 $\pm$ 0.86 | 1.39 $\pm$ 0.88 |
| $t_{1/2}$ (h) | 3.44 $\pm$ 4.26 | |
| $\lambda_z$ (h$^{-1}$) | 0.31 $\pm$ 0.15 | |
| CL/F (ng/h) | 1.11 $\pm$ 1.01 | 0.76 $\pm$ 0.23 |
| V/F (L) | 3.90 $\pm$ 2.90 | 2.36 $\pm$ 1.26 |
| Key as Table 8 | | |

From these preliminary results, it can be seen that comparison of the area under the plasma concentration curve during the 12 hours following the doses ($AUC_{0-12}$), a well-accepted measure of absorption, shows significant and surprisingly higher absorption of artemether when administered sublingually as a spray formulation as disclosed herein by comparison to oral tablet dosing.

For comparison of bioavailability of artemether via the sublingual spray route described herein with administration by oral tablets, we have calculated the F-values, commonly used to compare two dose regimes, generally A and B, for the artemether data, as follows:

$$F_{A-B} = \frac{AUC_A}{AUC_B} \frac{dose_B}{dose_A}$$

The results are as follows:

$$F_{T1-T4} = 1.67 \pm 0.60 \text{ (S.D.)}$$

$$F_{T2-T4} = 2.24 \pm 0.92 \text{ (S.D.)}$$

$$F_{T3\text{-}T4} = 2.09 \pm 0.69 \ (\text{S.D.})$$

This indicates that approximately between 1.7 and 2.2 times more artemether was absorbed when administered as a sublingual spray as described herein by comparison to oral administration by tablet, despite the oral dose being twice as large in the first instance. The indicative bioavailability by the sublingual route is therefore at least twice that by the oral route for equivalent doses.

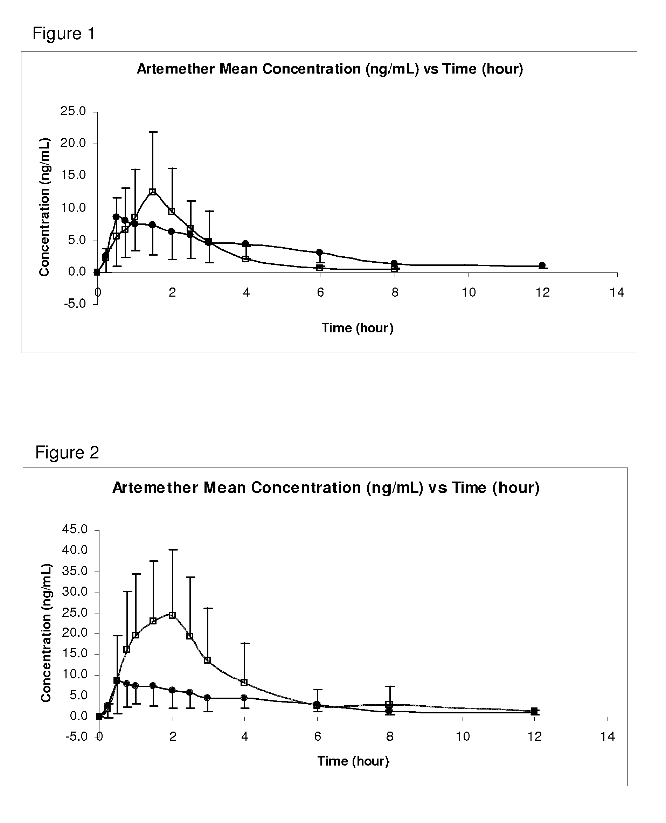

Inspection of the data of Tables 8-11, and Figures 1-12 also confirms this general finding for the primary active metabolite of artemether (dihydroartemesinin).

Avoidance of Autoinduction

It is known that both oral and rectal administration of artemesinins is associated with autoinduction of the drug metabolism in individuals (see e.g. Ashton M, Hai TN, Sy ND, Huong DX, Van Huong N, Nieu NT, Cong LD. "Artemisinin pharmacokinetics is time-dependent during repeated oral administration in healthy male adults.", Drug Metab Dispos. 1998; 26:25-7, and "Retrospective analysis of artemisinin pharmacokinetics: application of a semiphysiological autoinduction model ", Asimus and Gordi, Br. J Clin Pharmacol. 2007 June; 63(6): 758-762). As a result, systemically circulating artemesinin declines with each successive dose, thereby reducing the effectiveness of drug dosage regimes.

In confidential trials, the inventors have found that administration of artemesinins by the transmucosal sublingual route avoids such autoinduction, leading to consistent uptake and accumulating systemic concentration of the active drug metabolite, dihydroartemesinin, thereby providing significant advantage in administration by the sublingual route. A similar avoidance of autoinduction is expected with delivery by the transmucosal buccal or nasal route.

In confidential trials, volunteers followed the following treatment: A single administration of 30mg artemether sublingual spray 6mg/actuation on days 1 and 5 following an overnight fast, and twice daily administrations of 30mg artemether sublingual spray 3mg/actuation on days 2, 3,and 4 following a morning or evening meal. Blood samples were collected for pharmacokinetic analysis at the following time points:

Day 1: Predose, 0.25, 0.5, 0.75, 1, 1.5, 2, 2.5, 3, 4, 6, 8, and 12 h after dosing.
Days 2, 3, and 4: pre morning dose and 0.5, 1, 2 and 4 h after morning dose and pre evening dose and 1 hour after evening dose.

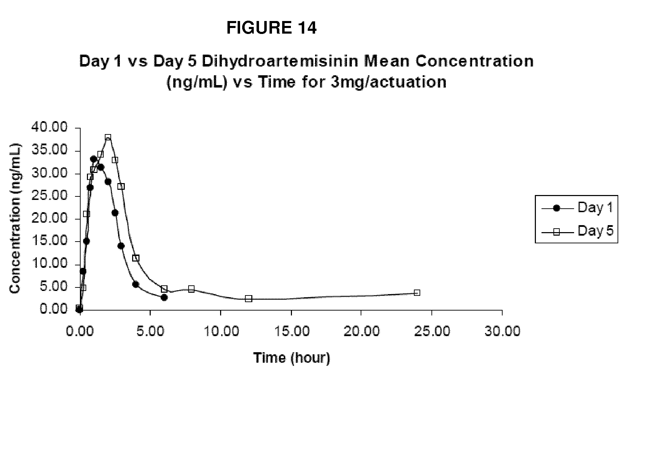

Day 5: Predose, 0.25, 0.5, 0.75, 1, 1.5, 2, 2.5, 3, 4, 6, 8, 12 h and 24 h after dosing. Pharmacokinetic analysis of plasma dihydroartemesinin on days 1 and 5 revealed an effectively identical response, indicating the lack of autoinduction. Plasma concentration curves are shown in Figure 14.

Figure Captions

Figure 1: Plot of mean plasma Artemether concentration vs time with standard deviation following a single sublingual administration of 15mg Artemether Sublingual Spray 3mg/actuation (T1) and single oral administration of 30mg Artemether Tablets 10 mg/tablet (T4). Mean $\pm$ SD (• = reference, T4 , D = test, T1)

Figure 2: Plot of mean plasma Artemether concentration vs time with standard deviation following a single sublingual administration of 30mg Artemether Sublingual Spray 3mg/actuation (T2) and single oral administration of 30mg Artemether Tablets 10 mg/tablet (T4). Mean $\pm$ SD (• = reference, T4 , D = test, T2)

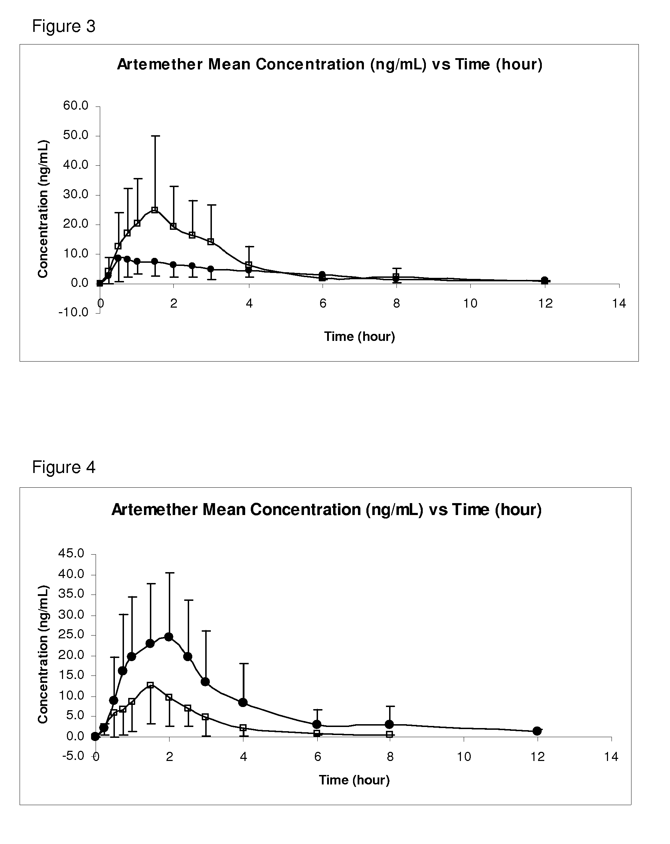

Figure 3: Plot of mean plasma Artemether concentration vs time with standard deviation following a single sublingual administration of 30mg Artemether Sublingual Spray 6mg/actuation (T3) versus single oral administration of 30mg Artemether Tablets 10 mg/tablet (T4). Mean $\pm$ SD (• = reference, T4 , D = test, T3)

Figure 4: Plot of mean plasma artemether concentration vs time with standard deviation following a single sublingual administration of 15mg Artemether Sublingual Spray 3mg/actuation (T1) versus single sublingual administration of 30mg Artemether Sublingual Spray 3mg/actuation (T2). Mean $\pm$ SD (• = reference, T2 , D = test, T1)

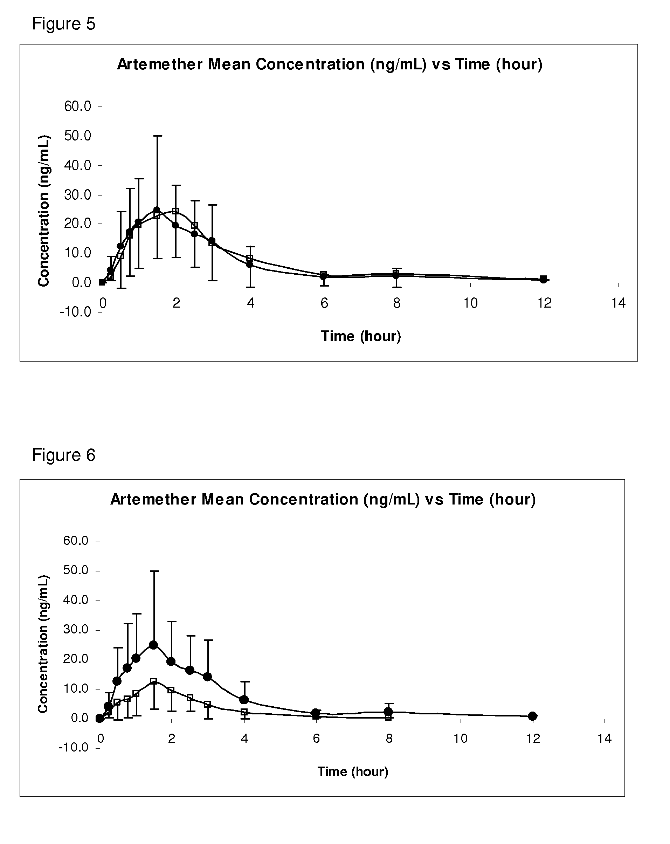

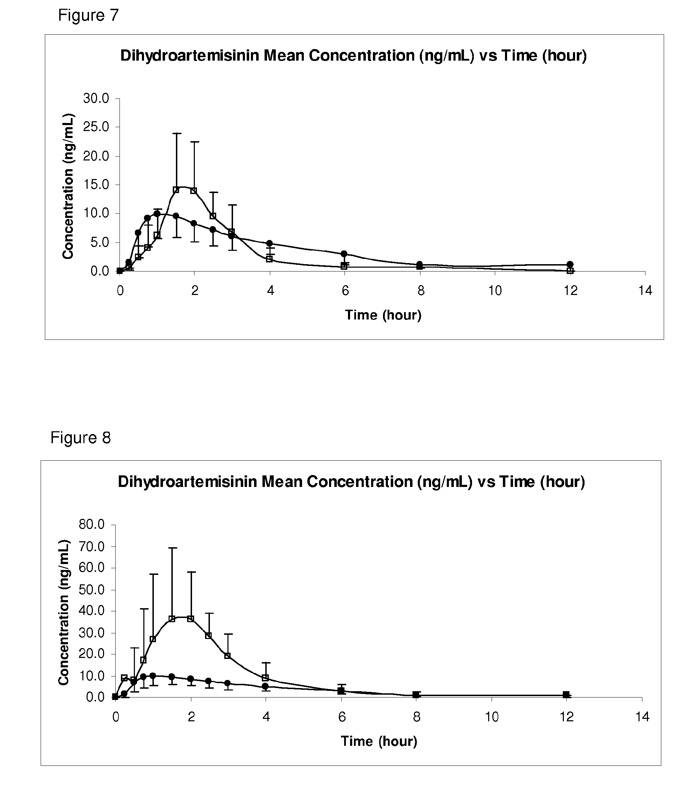

Figure 5: Plot of mean plasma Artemether concentration vs time with standard deviation following a single sublingual administration of 30mg Artemether Sublingual Spray 3mg/actuation (T2) versus single sublingual administration of 30mg Artemether Sublingual Spray 6mg/actuation (T3). Mean $\pm$ SD ($\bullet$ = reference, T3 , D = test, T2) Figure 6: Plot of mean plasma Artemether concentration vs time with standard deviation following a single sublingual administration of 15mg Artemether Sublingual Spray 3mg/actuation (T1) versus single sublingual administration of 30mg Artemether Sublingual Spray 6mg/actuation (T3). Mean $\pm$ SD ($\bullet$ = reference, T3 , D = test, T1) Figure 7: Plot of mean plasma Dihydroartemisinin concentration vs time with standard deviation following a single sublingual administration of 15mg Artemether Sublingual Spray 3mg/actuation (T1) and single oral administration of 30mg Artemether Tablets 10 mg/tablet (T4). Mean $\pm$ SD ($\bullet$ = reference, T4 , D = test, T1)

Figure 8: Plot of mean plasma Dihydroartemisinin concentration vs time with standard deviation following a single sublingual administration of 30mg Artemether Sublingual Spray 3mg/actuation (T2) and single oral administration of 30mg Artemether Tablets 10 mg/tablet (T4). Mean $\pm$ SD ($\bullet$ = reference, T4 , D = test, T2)

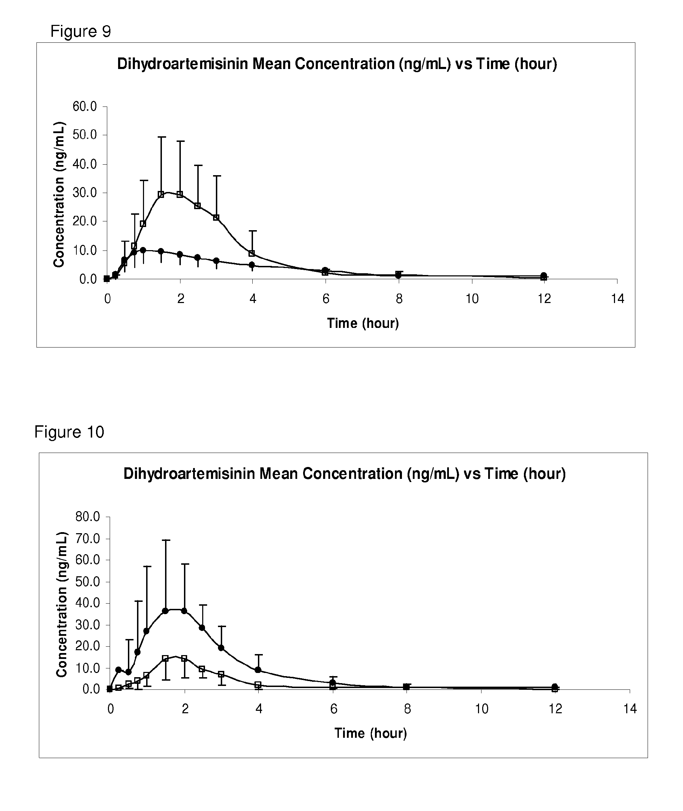

Figure 9: Plot of mean plasma Dihydroartemisinin concentration vs time with standard deviation following a single sublingual administration of 30mg Artemether Sublingual Spray 6mg/actuation (T3) versus single oral administration of 30mg Artemether Tablets 10 mg/tablet (T4). Mean $\pm$ SD ($\bullet$ = reference, T4 , D = test, T3)

Figure 10: Plot of mean plasma Dihydroartemisinin concentration vs time with standard deviation following a single sublingual administration of 15mg Artemether Sublingual Spray 3mg/actuation (T1) versus single sublingual administration of 30mg Artemether Sublingual Spray 3mg/actuation (T2). Mean $\pm$ SD ($\bullet$ = reference, T2 , D = test, T1)

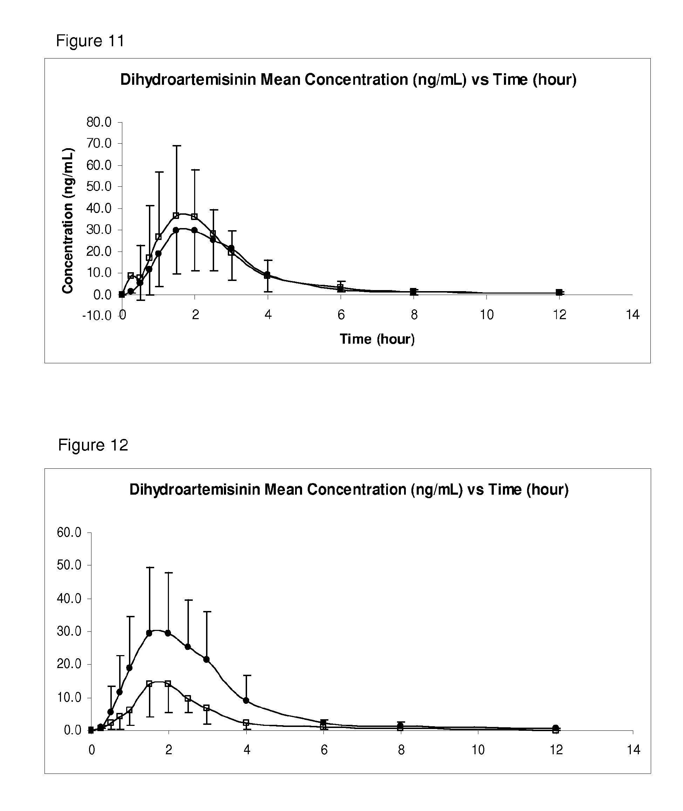

Figure 11: Plot of mean plasma Dihydroartemisinin concentration vs time with standard deviation following a single sublingual administration of 30mg Artemether Sublingual Spray 3mg/actuation (T2) versus single sublingual administration of 30mg Artemether Sublingual Spray 6mg/actuation (T3). Mean $\pm$ SD ($\bullet$ = reference, T3 , D = test, T2)

Figure 12: Plot of mean plasma Dihydroartemisinin concentration vs time with standard deviation following a single sublingual administration of 15mg Artemether Sublingual Spray 3mg/actuation (T1) versus single sublingual administration of 30mg Artemether Sublingual Spray 6mg/actuation (T3). Mean $\pm$ SD ($\bullet$ = reference, T3 , D = test, T1)

## Claims

1. A pharmaceutical composition comprising:

   artemesinin or derivative thereof; and
   a pharmaceutically-acceptable excipient selected from the group consisting of:

      medium chain length triglycerides;
      short chain triglycerides;
      omega-3-marine triglycerides; and
      fish oil, rich in omega-3-acids

   said composition formulated for transmucosal sublingual, buccal or nasal dosage.

2. A composition according to claim 1 consisting essentially of:

   artemesinin or derivative thereof; and
   one or more pharmaceutically-acceptable excipients selected from the group consisting of:

      medium chain length triglycerides;
      short chain triglycerides;
      omega-3-marine triglycerides; and
      fish oil, rich in omega-3-acids

   said composition formulated for transmucosal sublingual, buccal or nasal dosage.

3. A composition according to claim 1 consisting essentially of:

   artemesinin or derivative thereof; and

a pharmaceutically acceptable excipient consisting essentially of:

a triglyceride, liquid at 37°C; and
medium chain length triglycerides;
said composition formulated for transmucosal sublingual, buccal or nasal dosage.

4. A composition according to any preceding claim wherein said artemesinin or derivative thereof is selected from the group consisting of artemesinin, artemether, arteether, artesunate and dihydroartemesinin, and is preferably artemether or arteether.

5. A composition according to any preceding claim, substantially free of water and/or substantially free of ethanol.

6. A composition according to any preceding claim wherein said artemesinin or derivative thereof is present at a concentration of between 2 and 250 milligrams per gram of excipient.

7. A composition according to any preceding claim wherein said excipient comprises a medium chain triglyceride, said triglyceride comprising a minimum of 95 per cent of saturated fatty acids with between 6 and 12 carbon atoms, preferably wherein said triglyceride comprises a minimum of 95 per cent of saturated fatty acids with 8 and 10 carbon atoms.

8. A composition according to any preceding claim further comprising an essential oil such as menthol, vanillin or orange oil, lemon oil, clove oil, peppermint oil, spearmint oil.

9. A medicament delivery device containing a composition according to any preceding claim, said device adapted to deliver individual or successive doses of said composition, each individual or successive dose having a volume of less than 1000 microlitres.

10. A medicament delivery device containing a composition according to any one of claims 1 to 8, said device adapted to deliver individual or successive doses of said composition, each individual or successive dose containing no more than 80mg of artemesinin or derivative thereof, preferably no more than 10mg of artemesinin or derivative thereof.

11. A delivery device according to claim 9 or claim 10 wherein said device comprises a pump spray.

12. A delivery device according to claim 11 wherein said device is:

(a) manually-actuated; and/or
(b) adapted to produce a spray of composition having a mean droplet diameter greater than 20 microns.

13. A device for providing pharmaceutical doses comprising a container containing a pharmaceutical composition according to any one of claims 1 to 8, and valve means arranged to transfer doses of said pharmaceutical composition to the exterior of the container.

14. A composition according to any one of claims 1 to 8 for use in a method of treatment of the human or animal body by therapy, where in said treatment the composition is administered by the transmucosal sublingual, buccal or nasal route.

15. A composition for use according to claim 14 where in said treatment the composition is administered by spray.

Figure 1

Artemether Mean Concentration (ng/mL) vs Time (hour)

Figure 2

Artemether Mean Concentration (ng/mL) vs Time (hour)

Figure 3

**Artemether Mean Concentration (ng/mL) vs Time (hour)**

Figure 4

**Artemether Mean Concentration (ng/mL) vs Time (hour)**

Figure 5

**Artemether Mean Concentration (ng/mL) vs Time (hour)**

Figure 6

**Artemether Mean Concentration (ng/mL) vs Time (hour)**

Figure 7

Dihydroartemisinin Mean Concentration (ng/mL) vs Time (hour)

Figure 8

Dihydroartemisinin Mean Concentration (ng/mL) vs Time (hour)

Figure 9

Dihydroartemisinin Mean Concentration (ng/mL) vs Time (hour)

Figure 10

Dihydroartemisinin Mean Concentration (ng/mL) vs Time (hour)

Figure 11

**Dihydroartemisinin Mean Concentration (ng/mL) vs Time (hour)**

Figure 12

**Dihydroartemisinin Mean Concentration (ng/mL) vs Time (hour)**

## FIGURE 13: Representative chromatograms

a. Aerosol Formulation (20% v/v Ethanol; 80% v/v propellant)

b. Aerosol Formulation (50% v/v Ethanol; 50% v/v propellant)

c. Pump Spray formulation (Ethanol solvent)

d. Pump Spray formulation (Miglyol solvent)

FIGURE 14

Day 1 vs Day 5 Dihydroartemisinin Mean Concentration (ng/mL) vs Time for 3mg/actuation

Europäisches
Patentamt

European
Patent Office

Office européen
des brevets

# EUROPEAN SEARCH REPORT

Application Number

EP 14 16 1340

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 1 650 854 A (GUANGZHOU ZHONGSHENG BIOTECHNO [CN]) 10 August 2005 (2005-08-10) * the whole document * | 1-4,7, 14,15 | INV. A61K9/00 A61K31/357 A61K47/44 A61P33/06 |
| X | US 5 057 501 A (THORNFELDT CARL R [US]) 15 October 1991 (1991-10-15) * abstract * * column 1, last paragraph - column 2, paragraph second * * column 5 * * claim 21 * | 1-4 | |
| X | US 6 306 896 B1 (SCHEIWE MAX WERNER [DE]) 23 October 2001 (2001-10-23) * abstract * * column 2 * * column 6; examples 1-9 * | 1,4 | |
| A | US 2003/203875 A1 (HARTELL MARK G [US] ET AL) 30 October 2003 (2003-10-30) * page 6, left-hand column, last paragraph - right-hand column, paragraph FIRST * * claims 12-17 * | 1-15 | **TECHNICAL FIELDS SEARCHED (IPC)** A61K A61P |
| A | WO 2007/043057 A (YISSUM RES DEV CO [IL]; TOUITOU ELKA [IL]; GODIN BIANA [IL]; DUCHI SHA) 19 April 2007 (2007-04-19) * page 33; example 12 * * claims 40-45 * | 1-15 | |
| A | US 2004/038933 A1 (KANEKO YUTARO [JP] ET AL) 26 February 2004 (2004-02-26) * page 1, left-hand column, paragraph FIRST * * page 3, right-hand column, paragraph 5 * | 1-15 | |

-/--

The present search report has been drawn up for all claims

| Place of search | Date of completion of the search | Examiner |
|---|---|---|
| Munich | 30 April 2014 | Damiani, Federica |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

Europäisches
Patentamt

European
Patent Office

Office européen
des brevets

# EUROPEAN SEARCH REPORT

Application Number

EP 14 16 1340

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | TOUITOU ET AL: "Treatment of malaria in a mouse model by intranasal drug administration", INTERNATIONAL JOURNAL OF PARASITOLOGY, PERGAMON PRESS, GB, vol. 36, no. 14, 19 November 2006 (2006-11-19), pages 1493-1498, XP005774092, ISSN: 0020-7519 * page 1494, left-hand column, paragraph 3 * * page 1497, right-hand column, paragraph 2 * ----- | 1-15 | |

TECHNICAL FIELDS
SEARCHED      (IPC)

The present search report has been drawn up for all claims

| Place of search | Date of completion of the search | Examiner |
|---|---|---|
| Munich | 30 April 2014 | Damiani, Federica |

EPO FORM 1503 03.82 (P04C01)

**ANNEX TO THE EUROPEAN SEARCH REPORT
ON EUROPEAN PATENT APPLICATION NO.**                   EP 14 16 1340

This annex lists the patent family members relating to the patent documents cited in the above-mentioned European search report.
The members are as contained in the European Patent Office EDP file on
The European Patent Office is in no way liable for these particulars which are merely given for the purpose of information.

30-04-2014

| Patent document cited in search report | | Publication date | Patent family member(s) | | Publication date |
|---|---|---|---|---|---|
| CN 1650854 | A | 10-08-2005 | NONE | | |
| US 5057501 | A | 15-10-1991 | NONE | | |
| US 6306896 | B1 | 23-10-2001 | AT | 352297 T | 15-02-2007 |
| | | | AU | 8796498 A | 24-05-1999 |
| | | | CH | 692321 A5 | 15-05-2002 |
| | | | EP | 1028722 A1 | 23-08-2000 |
| | | | JP | 4750270 B2 | 17-08-2011 |
| | | | JP | 2001521894 A | 13-11-2001 |
| | | | PL | 340266 A1 | 29-01-2001 |
| | | | US | 6306896 B1 | 23-10-2001 |
| | | | WO | 9922727 A1 | 14-05-1999 |
| US 2003203875 | A1 | 30-10-2003 | AU | 2003219963 A1 | 22-09-2003 |
| | | | US | 2003203875 A1 | 30-10-2003 |
| | | | US | 2005187189 A1 | 25-08-2005 |
| | | | US | 2006229279 A1 | 12-10-2006 |
| | | | WO | 03075904 A2 | 18-09-2003 |
| WO 2007043057 | A | 19-04-2007 | AT | 544444 T | 15-02-2012 |
| | | | CN | 101325944 A | 17-12-2008 |
| | | | EP | 1933809 A2 | 25-06-2008 |
| | | | ES | 2385513 T3 | 26-07-2012 |
| | | | JP | 5362360 B2 | 11-12-2013 |
| | | | JP | 2009511571 A | 19-03-2009 |
| | | | PT | 1933809 E | 26-04-2012 |
| | | | SI | 1933809 T1 | 31-08-2012 |
| | | | WO | 2007043057 A2 | 19-04-2007 |
| US 2004038933 | A1 | 26-02-2004 | AU | 9597901 A | 29-04-2002 |
| | | | EP | 1329452 A1 | 23-07-2003 |
| | | | US | 2004038933 A1 | 26-02-2004 |
| | | | WO | 0232905 A1 | 25-04-2002 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6306896 B

**Non-patent literature cited in the description**

- **WOODROW et al.** *Postgrad. Med.J.,* 2005, vol. 81, 71-78
- **NAVARATNAM et al.** *Clin Pharmacokinet,* October 2000, vol. 39 (4), 255-270
- **ASHTON M ; HAI TN ; SY ND ; HUONG DX ; VAN HUONG N ; NIEU NT ; CONG LD.** Artemisinin pharmacokinetics is time-dependent during repeated oral administration in healthy male adults. *Drug Metab Dispos,* 1998, vol. 26, 25-7
- **ASIMUS ; GORDI.** Retrospective analysis of artemisinin pharmacokinetics: application of a semiphysiological autoinduction model. *Br. J Clin Pharmacol.,* June 2007, vol. 63 (6), 758-762